# EUROPÄISCHE PATENTSCHRIFT

(11) **EP 0 534 901 B1**
(45) Veröffentlichungstag und Bekanntmachung des Hinweises auf die Patenterteilung: **21.02.1996**
(21) Anmeldenummer: 92810675.6
(22) Anmeldetag: 03.09.1992
(51) Int. Cl.: D02J 1/22, G01N 33/36, G01N 27/06

(54) **Streckkammeranordnung**
Stretching-chamber assembly
Assemblage de chambre d'étirage

(30) Priorität: 23.09.1991 CH 2819/91
(43) Veröffentlichungstag der Anmeldung: 31.03.1993
(73) Patentinhaber: MASCHINENFABRIK RIETER AG, CH-8406 Winterthur (CH)
(72) Erfinder: Graf, Felix, CH-8400 Winterthur (CH); Rauchegger, Günther, CH-8408 Winterthur (CH)
(74) Vertreter: Frei, Alexandra Sarah

(56) Entgegenhaltungen:
- GB-A- 2 217 848
- GB-A- 2 230 098
- US-A- 3 045 315

## Beschreibung

Die Erfindung liegt auf dem Gebiete der Textiltechnik und betrifft eine Streckkammeranordnung gemäss dem Oberbegriff der unabhängigen Ansprüche 1 und 10. In der beispielsweise aus der US-A- 3 045 315 bekannten Streckkammeranordnung werden synthetische Filamente unter on-line kontrollierten Verhältnissen hydrodynamisch gestreckt.

Synthetische Filamente, insbesondere linear polymere Filamente (Glattgarne) werden nach der Extrusion gestreckt, um die Moleküle in Garnrichtung zu orientieren. Es hat sich als vorteilhaft erwiesen, für eine derartige Streckung Flüssigkeitsbäder zu verwenden, in denen die Streckung durch eine Kombination von hydrodynamischer und mechanischer Bremsung bewirkt wird. Insbesondere hat es sich als vorteilhaft erwiesen, die Streckung in quasi geschlossenen Kammern (Streckkammern) durchzuführen, in denen eine Kammerflüssigkeit in einer dem Faden entgegengesetzten Richtung fliesst. Dabei zeigt es sich, dass die Bremswirkung der Kammerflüssigkeit, die von der Eintauchlänge des Fadens in der Flüssigkeit, von der Kammergeometrie und von den Eigenschaften der Kammerflüssigkeit abhängig ist, und die Temperatur sehr wichtige Verfahrensparameter sind.

Die US-PS 3045315 zeigt und beschreibt eine Vorrichtung zum Strecken eines Filamentfadens, bei welchem der Filamentfaden während des Streckprozesses durch eine mit einer Flüssigkeit gefüllte Kammer geführt wird. Dabei wird Flüssigkeit aus einem Tank in die Kammer gepumpt und der Überlauf aus der Kammer gelangt wieder in den Tank. Hier bildet die Flüssigkeit in der Kammer nicht einen Widerstand für den Filamentfaden, sondern lediglich eine Benetzungs- und Erwärmungsmöglichkeit, indem der Filamentfaden um einen in die Flüssigkeit der Kammer eingetauchten Streckstift gelenkt wird. Die vom Filamentfaden mittels Umlenkung an der Oberfläche des Filmentfadens abgeschiedene Flüssigkeit gelangt ebenfalls in den Tank zurück. Der eigentliche Streckprozess geschieht jedoch zwischen einer Streckrolle vor und einer Streckrolle nach der Kammer.

In der EP-PA0384886 derselben Anmelderin, die hier als bekannt vorausgesetzt wird, sind entsprechende Streckverfahren und Streckkammern zur Durchführung der Verfahren beschrieben.

Diese Streckkammern sind meist derart angeordnet, dass der Faden sie im wesentlichen in Richtung Schwerkraft durchläuft, wobei die Kammerflüssigkeit gegenläufig von unten zu- und oben abgeführt wird. Ein Teil der Flüssigkeit wird vom schnell laufenden Faden aus der Kammer mitgerissen und muss anschliessend mit entsprechenden Mitteln vom Faden entfernt werden. Die Geometrie der Kammer kann anwendungsspezifisch einstellbar sein. Als Kammerflüssigkeit wird meist eine Emulsion eingesetzt, deren Eigenschaften primär von der Zusammensetzung abhängig sind. Die Temperatur und die Zusammensetzung der Kammerflüssigkeit werden in einem Tank auf die anwendungsspezifischen Werte eingestellt, von wo die Flüssigkeit in einem geschlossenen Kreislauf durch die Kammer gefördert wird. Die Eintauchlänge des Fadens in der Kammerflüssigkeit entspricht im einfachsten Falle der Länge der Kammer, kann aber durch Nacheinanderschalten von mehreren Kammern oder durch Eintrittsöffnungen für den Faden an verschiedenen Stellen der Kammer eventuell variiert werden. Die Durchflussgeschwindigkeit der Kammerflüssigkeit durch die Kammer ist an sich ein nur untergeordneter Verfahrensparameter, da sie sehr viel kleiner ist als die Fadengeschwindigkeit in entgegengesetzter Richtung und als die Flüssigkeitsgeschwindigkeiten, die durch die Pumpwirkung des Fadens als interne Zirkulation erzeugt werden. Es ist aber sehr wichtig, dass die Kammer ständig den gleichen Füllgrad aufweist, also beispielsweise immer voll ist, dass also die vom Faden mitgerissene Kammerflüssigkeit kontinuierlich ersetzt wird, und dass die Kammertemperatur in gegebenen Grenzen konstant ist. Dafür ist eine minimale Durchflussgeschwindigkeit unabdingbar, die ihrerseits eine Funktion der Garngeschwindigkeit ist.

Störungen im Betrieb von derartigen Streckkammern entstehen, wenn bedingt durch Bedienungsfehler, Verschmutzung oder externe Störungen die Bremswirkung und/oder die Temperatur von ihrem vorgegebenen Wert abweichen.

Wird eine derartige Streckkammer mit einer Emulsion von Spin-Finish in Wasser betrieben, kann sie neben ihrer Funktion als Streckkammer gleichzeitig für das Aufbringen von Spin-Finish auf den Faden dienen, also die Funktion von Präparations-Auftragelementen übernehmen. Damit der Faden nach dem Austritt aus der Kammer und dem Durchlaufen einer Vorrichtung zur Abscheidung der überschüssigen Flüssigkeit eine für die Weiterverarbeitung optimale Menge von Spin-Finish mit sich trägt, ist es wichtig, die Konzentration des Spin-Finish in der Emulsion in engen Grenzen konstant zu halten. Diese Konzentration kann sich aber vor allem durch Wasserverdampfung bei relativ hohen Kammertemperaturen während dem Betrieb verändern.

Um einen Streckkammer-Betrieb mit konstanten Flüssigkeitsparametern und frei von Störungen auch der auf die Streckkammer folgenden Vorrichtungen zu gewährleisten, wäre es wünschenswert, die Kammerflüssigkeit zu kontrollieren und entsprechende Messgrössen in entsprechende Stellgrössen zur Verschiebung der Verfahrensparameter oder in Alarmsignale beim Überschreiten von Alarmgrenzen zu verarbeiten. Es würde dadurch auch möglich, die Streckkammeranordnung in einen vollautomatischen Produktionsprozess einzuordnen.

Die Erfindung stellt sich nun die Aufgabe, eine Streckkammeranordnung zu schaffen, mit Hilfe derer die Kammerflüssigkeit on-line derart kontrolliert werden kann, dass Schwankungen in der Fadenqualität und Betriebsstörungen bedingt durch Schwankungen in der Bremswirkung der Kammerflüssigkeit möglichst vermieden werden. Ferner soll die Anordnung die Funktion übernehmen können, den Faden für die Weiterverarbeitung mit einer in engen Grenzen konstanten Menge von Spin-Finish zu versehen.

Diese Aufgabe wird gelöst durch die Streckkammeranordnung gemäss dem kennzeichnenden Teil der unabhängigen Ansprüche. Es handelt sich dabei um eine Anordnung mit einer Streckkammer, einem Flüssigkeitskreislauf mit Zuführung zur Kammer, Abführung aus der Kammer, Tank und Pumpmitteln und Mitteln zur on-line-Kontrolle des Flüssigkeitsniveaus im Überlauf der Kammer und der Qualität der Kammerflüssigkeit durch Messung ihres elektrischen Widerstandes.

Die erfindungsgemässe Streckkammer soll anhand der folgenden Figuren detailliert beschrieben werden. Dabei zeigen:
- **Figur 1**: ein Schema einer ersten beispielhaften Ausführungsform der erfindungsgemässen Streckkammeranordnung;
- **Figur 2**: ein Schema einer weiteren beispielhafte Ausführungsform;
- **Figur 3**: ein beispielhaftes Funktionsschema für eine Steuereinheit für die Ausführungsform gemäss der Figur 2;
- **Figur 4**: eine beispielhafte Anordnung des Messelementes im Überlauf der Streckkammer;
- **Figur 5**: eine weitere beispielhafte Anordnung des Messelementes im Überlauf der Streckkammer;
- **Figur 6**: Verlauf des Messsignales für die Anordnung gemäss Figur 5.

Die Bremswirkung der Kammerflüssigkeit ist im wesentlichen von der Eintauchlänge des Fadens in der Kammerflüssigkeit, also bei im wesentlichen senkrechtem Fadenverlauf vom Flüssigkeitsniveau in der Kammer und von den Eigenschaften der Kammerflüssigkeit abhängig, die Temperatur vom Durchlauf in der Kammer und von der Fadengeschwindigkeit, wenn von einem Flüssigkeitstank mit konstant gehaltener Flüssigkeitstemperatur ausgegangen wird.

Die Kammerflüssigkeit ist in den meisten Fällen eine Emulsion, deren elektrischer Widerstand direkt von der Zusammensetzung abhängig ist und der einfach gemessen werden kann. Es wird beispielsweise der Strom zwischen zwei, in die Flüssigkeit eintauchenden, voneinander beabstandeten, mit einer Spannungsdifferenz beschalteten Elektroden gemessen, wobei die Funktion der einen Elektrode auch von einem geerdeten Teil der Vorrichtung übernommen werden kann. Die Messgrösse ist abhängig von der Messanordnung (Messspannung, Abstand der Elektroden und Fläche der Elektroden) und vom spezifischen Widerstand des Mediums zwischen den beiden Elektroden, der seinerseits eine Funktion der Eigenschaften dieses Mediums ist, beispielsweise der Konzentration des öligen Anteils in einer wässerigen Emulsion. Eine derartige Messung soll nun in der erfindungsgemässen Streckkammeranordnung dazu angewendet werden, das Niveau der Kammerflüssigkeit im Überlauf der Kammer und die Qualität der Kammerflüssigkeit zu kontrollieren.

Dazu wird einerseits im Tank der Kammerflüssigkeit, in dem auch die Zusammensetzung und die Temperatur der Kammerflüssigkeit eingestellt werden, ein Sensor zur Messung des elektrischen Widerstandes der Kammerflüssigkeit angeordnet. Andererseits ist der Überlauf der Kammer derart ausgestaltet, dass das darin durch die in den Tank zurückfliessende Kammerflüssigkeit gebildetc Niveau empfindlich von der Durchflussgeschwindigkeit abhängig ist.

In diesem Überlauf ist ein zweiter Sensor zur Messung des elektrischen Widerstandes angeordnet, und zwar derart, dass die Grösse des durch die Flüssigkeit benetzten Teils der Elektroden vom Stand des Niveaus abhängig ist. Signalleitungen von den beiden Sensoren werden auf eine Steuerung geführt, die die Signale verarbeitet und daraus Steuer- und/oder Alarmsignale generiert.

Mit einer derartigen Anordnung ist es möglich, während dem Betrieb der Streckkammer das Flüssigkeitsniveau in der Kammer zu überwachen und bei nicht tolerierbar niedrigem oder hohem Niveau über entsprechende Stellglieder den Durchfluss derart zu regulieren, dass sich ein vorgegebenes Niveau einstellt, oder aber einen Alarm zu generieren. Ebenso ist es möglich, die Eigenschaften der Kammerflüssigkeit im Tank und/oder in der Kammer zu überwachen, woraus Rückschlüsse auf deren Qualität, insbesondere Zusammensetzung oder Mischungsgrad gezogen werden können. Die Anordnung macht es auch möglich, die Streckkammer erst freizugeben, wenn die Kammerflüssigkeit die vorgegebenen Eigenschaften (Niveau, Zusammensetzung, Mischungsgrad) hat. Bei Schwankungen in der Fadengeschwindigkeit ist es möglich, das Flüssigkeitsniveau trotzdem konstant zu halten, bei Wasserverlusten aus einer emulgierten Kammerflüssigkeit durch Verdampfung ist es möglich die Zusammensetzung der Emulsion konstant zu halten.

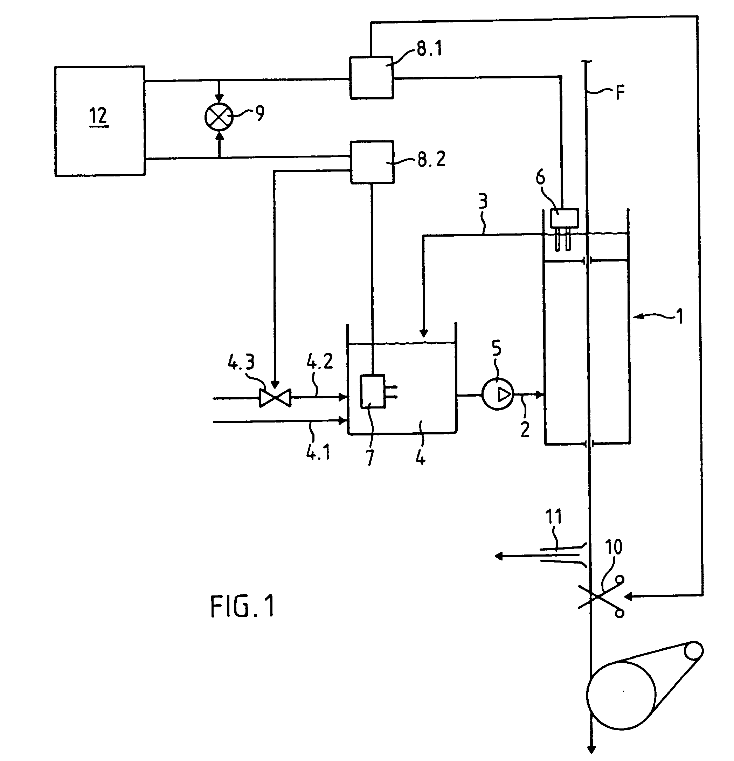

**Figur 1** zeigt ein Schema einer ersten beispielhaften Ausführungsform der erfindungsgemässen Streckkammeranordnung. Sie weist eine Streckkammer 1 mit einer Flüssigkeitszuführung 2 und einer Flüssigkeitsabführung 3, die die Flüssigkeit aus dem Überlauf 1.1 der Kammer abführt, auf. Die Kammerflüssigkeit wird von der Flüssigkeitsabführung 3 in einen Tank 4 geleitet und aus dem Tank 4 durch eine Pumpe 5 wieder der Kammer zugeführt. Der Tank 4 besitzt eine Flüssigkeitszuspeisung 4.1 und eine Wasserzuspeisung 4.2, durch die fertig gemischte Kammerflüssigkeit, bzw. reines Wasser bei Bedarf in den Tank nachgespeist werden. Die Zuspeisung der Kammerflüssigkeit wird über eine Füllstandsüberwachung oder ähnliches (in der Figur nicht dargestellt) gesteuert. Die Wasserzuspeisung wird durch ein Dosierventil 4.3 kontrolliert.

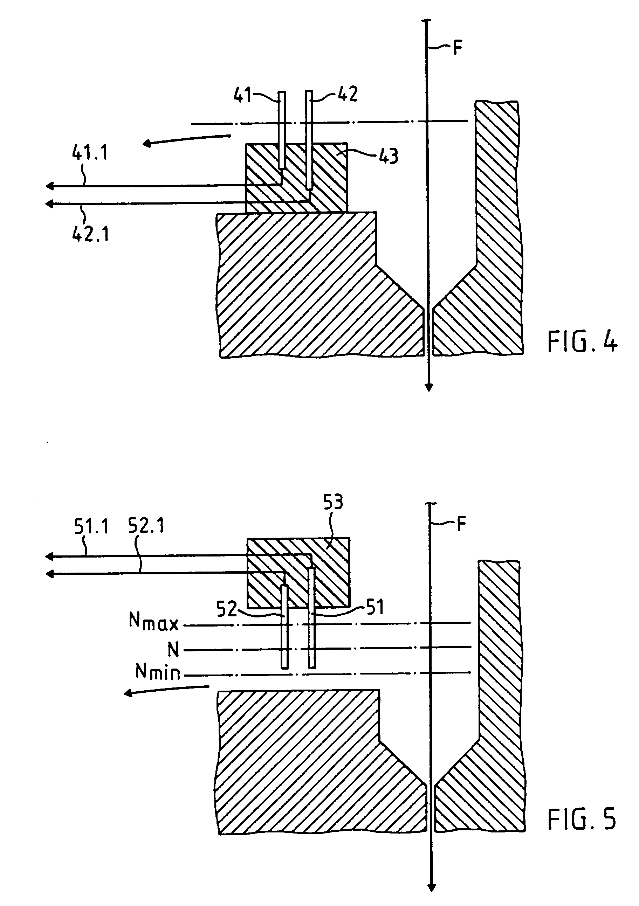

Im Überlauf 1.1 der Kammer 1 ist ein Messelement 6 angeordnet, das mit zwei mindestens teilweise in die Flüssigkeit eintauchenden Elektroden den elektrischen Widerstand der Flüssigkeit misst. Die Elektroden des Messelementes 6 ragen von unten oder von oben derart in die Flüssigkeit des Überlaufes, dass bei höherem Niveau ein grösserer Teil der Elektrodenfläche benetzt ist. Bei konstanter Qualität (Zusammensetzung) der Kammerflüssigkeit ist der durch die Elektroden fliessende Strom direkt abhängig vom, bei entsprechender Anordnung proportional zum Niveau im Überlauf. Das Messsignal des Messelementes 6 wird auf einen Toleranzbandumsetzer 8.1 geleitet und durch diesen mit einem vorgebbaren Toleranzband verglichen. Liegt der Messwert ausserhalb des im Toleranzbandumsetzer vorgegebenen Toleranzbandes, generiert dieser ein Signal, mit dem beispielsweise eine Signallampe 9 angesteuert wird. Daneben wird das Signal einer Fadenschere 10 zugeführt, die mit einer sinnvollen Verzögerung den Faden am Austritt der Kammer schneidet, sodass er in eine Abfallabsaugung 11 abgesaugt wird. Das Signal des Toleranzbandumsetzers kann auch einem Prozelssleitsystem 12 zugeführt und für weitere Steuerungen, beispielsweise der Pumpe 5 weiterverarbeitet werden. Beispielhafte Anordnungen von Messelementen 6 im Überlauf 1.1 der Kammer werden noch im Zusammenhang mit den Figuren 4 und 5 beschrieben.

Auch im Tank 4 ist ein Messelement 7 angeordnet, das den elektrischen Widerstand der Flüssigkeit misst. Dieses Messelement ist derart angeordnet, dass seine Messung nicht vom Flüssigkeitsstand im Tank abhängig und dadurch nur eine Funktion der Zusammensetzung der Flüssigkeit ist. Das Messsignal des Messelementes 7 wird ebenfalls auf einen Toleranzbandumsetzer 8.2 geleitet und von diesem mit einem vorgegebenen Toleranzband verglichen. Liegt der Messwert ausserhalb des Toleranzbandes, wird beispielsweise die Signallampe 9 und das Dosierventil 4.3 angesteuert. Auch das Signal des Toleranzbandumsetzers 8.2 kann dem Prozessleitsystem 12 zugeführt und für weitere Steuerungen weiterverarbeitet werden. Als Messelement 7 kann ein handelsübliches Messgerät zur Messung des elektrischen Widerstandes einer Flüssigkeit zur Anwendung kommen.

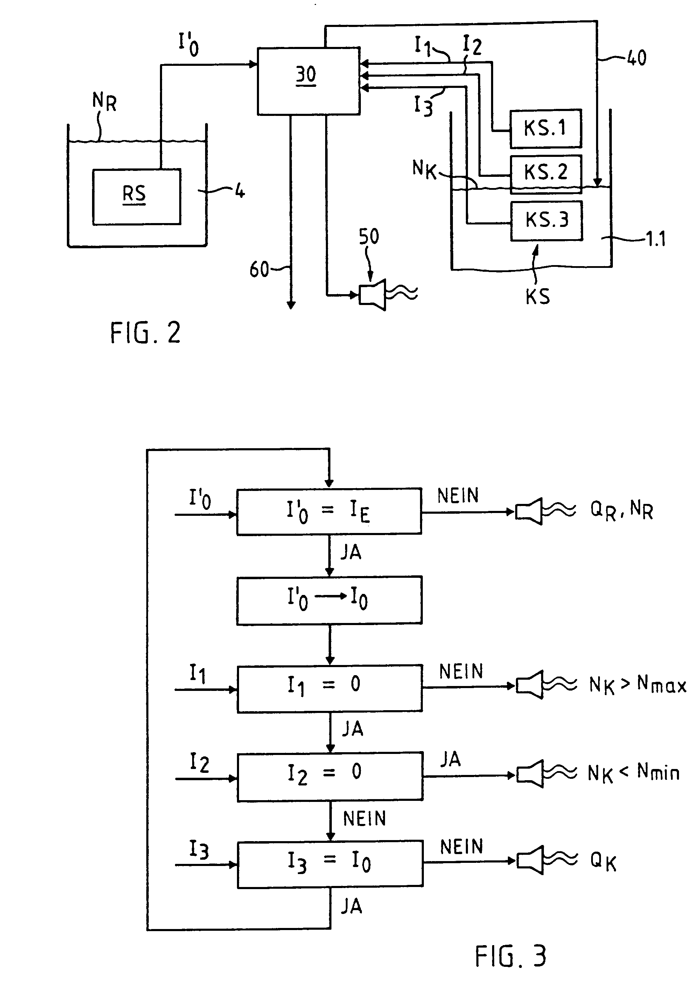

**Figur 2** zeigt schematisch eine weitere, beispielhafte Ausführungsform der Anordnung der Messelemente im Überlauf 1.1 der Kammer und im Tank 4. Diese Ausführungsform erlaubt zusätzlich zur Überwachung des Niveaus im Überlauf und der Flüssigkeitsqualität im Tank auch eine Überwachung der Flüssigkeitsqualität im Überlauf. Die linke Seite der Figur zeigt schematisch den Tank 4 mit einem darin unter der Flüssigkeitsoberfläche N_{R} angeordneten Messelement RS, mit dem der elektrische Widerstand der Kammerflüssigkeit gemessen wird. Der vom Messelement RS beispielsweise als Messsignal abgenommene Strom I'₀ ist abhängig von der Anordnung des Messelementes RS, von den Eigenschaften Q_{R} der Flüssigkeit im Tank, die im Falle einer Emulsion hauptsächlich von der Zusammensetzung, vom Mischungsgrad und der Temperatur abhängig sind. Das Messsignal I'₀ = f(RS,Q_{R}) wird einer Steuereinheit 30 zugeführt. Solange das Messelement RS ganz eingetaucht ist, ist das Messsignal nicht vom Niveau N_{R} abhängig.

Die rechte Seite der Figur zeigt schematisch das im Überlauf 1.1 der Streckkammer angeordnete Messelement KS, dessen mindestens eine Elektrode aufgeteilt ist in mindestens drei Teilelektroden KS.1, KS.2, und KS.3, die derart angeordnet sind, dass das zu kontrollierende Niveau N_{K} der Kammerflüssigkeit parallel zur Trennfläche der einzelnen Teilelektroden verläuft und dass die mittlere Teilelektrode KS.2 sich auf der Höhe des angestrebten Flüssigkeitsstandes befindet. Alle Teilelektroden sind mit einer Steuereinheit 30 verbunden und liefern Teilmesswerte I₁, I₂, bzw. I₃, die abhängig sind von der Sensoranordnung KS, die vorteilhafterweise für alle Teilelektroden dieselbe ist, und von den Eigenschaften des gemessenen Mediums, die im Falle des Überlaufes vom Niveau N_{K} der Kammerflüssigkeit (je nach Eintauchtiefe der entsprechenden Teilelektrode ist das Medium Luft oder Kammerflüssigkeit) und von den Eigenschaften Q_{K} der Kammerflüssigkeit im Überlauf 1.1 bestimmt werden, das heisst: I_{1,2,3} = f(KS,Q_{K},N_{K}).

Die Steuereinheit 30 kann mit einem Stellglied verbunden sein (schematisch als Pfeil 40 dargestellt), das die Durchlaufmenge der Flüssigkeit durch die Kammer und damit das Niveau im Überlauf 1.1 bestimmt, beispielsweise mit einer entsprechenden Steuerung der Flüssigkeitspumpe des Flüssigkeitskreislaufes (in der Figur nicht dargestellt). Zusätzlich kann die Steuereinheit mit einem Alarmsystem 50 verbunden sein, das beim Überschreiten eines Grenzwertes aktiviert wird. Die Steuereinheit 30 kann auch mit einer übergeordneten Steuerung verbunden sein (Pfeil 60), der sie dadurch gewisse Steuersignale übermitteln kann.

**Figur 3** zeigt ein Schema zur Erläuterung der Funktion einer beispielhaften Ausführungsform der Steuereinheit 30. Es gilt für eine Anordnung gemäss dem Schema der Figur 2, also mit drei Teilelektroden im Überlauf der Kammer, wobei der Soll-Niveaustand im Bereich der mittleren Teilelektrode KS.2 ist. Qualität und Temperatur der Kammerflüssigkeit im Überlauf der Kammer soll zu jeder Zeit der Qualität und der Temperatur im Tank entsprechen, die ihrerseits über ein entsprechend vorgegebenes Toleranzband (Eichwert) des elektrischen Widerstandes kontrolliert werden sollen.

Die Steuereinheit fragt nacheinander die einzelnen Messwerte I'₀,I₁,I₂ und I₃ ab und verarbeitet sie wie folgt:

Der Messwert I'₀ wird mit einem entsprechenden, vorgegebenen Eichwert I_{E} verglichen. Stimmen die Werte nicht in vorgegebenen Grenzen überein, schliesst die Steuereinheit auf falsche Eigenschaften Q_{R} der Kammerflüssigkeit im Tank (Zusammensetzung, Mischungsgrad, Temperatur) oder zu tiefes Niveau N_{R} im Tank und generiert einen entsprechenden Alarm oder entsprechende Signale für andere Kontrolleinheiten. Stimmt der Wert I'₀ mit dem Wert I_{E} überein, wird er reduziert auf einen Wert I_{0'} der einer Sensoranordnung gemäss den Teilelektroden des Sensors KS in Fläche und Abstand der Elektroden entspricht und der dadurch mit den Messwerten der Teilelektroden KS.1/2/3 direkt verglichen werden kann.

Der Messwert I₁ der Teilelektrode KS.1 wird mit einem sehr tief angesetzten Grenzwert verglichen, das heisst, es wird kontrolliert, ob die Teilelektrode KS.1 oberhalb des Flüssigkeitsniveaus N_{K} liegt. Ist dies nicht der Fall, generiert die Steuereinheit einen Alarm für zu hohes Niveau im Überlauf der Kammer oder ein entsprechendes Steuersignal für beispielsweise die Flüssigkeitspumpe.

Der Messwert I₂ der Teilelektrode KS.2 wird ebenfalls mit demselben Grenzwert verglichen. Ist auch ihr Signal sozusagen gleich Null, bedeutet das, dass auch die Teilelektrode KS.2 ganz oberhalb des Flüssigkeitsspiegels liegt. In diesem Falle erzeugt die Steuereinheit ein Alarmsignal für zu tiefes Niveau im Überlauf der Kammer oder ein entsprechendes Steuersignal.

Hat die Verarbeitung der beiden Messsignale I₁ und I₂ ein richtiges Niveau N_{K} ergeben, wird der Messwert I₃ mit dem Wert I₀ verglichen. Stimmen die Werte nicht in vorgegebenen Grenzen überein, wird ein Alarm generiert der aussagt, dass die Eigenschaften Q_{K} der Kammerflüssigkeit im Überlauf nicht denen der Kammerflüssigkeit im Tank entsprechen, dass also deren Zusammensetzung, deren Mischungsgrad oder/oder deren Temperatur nicht den vorgegebenen Werten entsprechen.

Zusätzlich kann die Steuereinheit 30 Funktionen ausüben, die sich auf die Anfahrphase beziehen. Sie kann beispielsweise den Messwert I'₀ während der Anfahrphase überwachen und ein entsprechendes Signal generieren, sobald dieser mit einem entsprechenden Eichwert I_{E} übereinstimmt, wodurch der Start für die ganze Anlage freigegeben wird. Besitzt die Steuereinheit den Eichwert I_{E} nicht, kann sie zuwarten, bis der Messwert I'₀ in vorgegebenen Grenzen konstant ist, kann dann den Messwert I'₀ als Eichwert I_{E} speichern und den Start freigeben.

Es ist nicht zwingend, dass die drei Teilelektroden KS.1/2/3 dieselbe Fläche und denselben Abstand zur Referenzelektrode haben, das heisst es kann sich auch um drei separate, verschiedene Sensoren handeln. In einem solchen Falle müssen die Messwerte I₁, I₂ und I₃ vor einem Vergleich miteinander, mit dem Messwert I'₀ oder mit einem Eichwert I_{E} auf Bedingungen eines Standardsensors reduziert werden.

Das Messelement im Überlauf der Kammer kann auch zur Erhöhung der Messgenauigkeit mehr als drei Teilelektroden aufweisen. Zur Erhöhung der Genauigkeit der Messung des Niveaus N_{K} kann auch aus dem Messsignal I₂ die genaue Lage des Niveaus durch Bildung des Quotienten I₂/I₃ bestimmt werden.

Es sind auch Anordnungen denkbar, bei denen das Messelement im Tank fehlt und die Steuereinheit die Messsignale der Teilelektroden mit vorgegebenen Toleranzbändern vergleicht. Eine derartige Anordnung misst dann mit einer Messanordnung an einer Stelle sowohl das Niveau als auch die Qualität der Flüssigkeit, liefert aber nur ein sinnvolles Resultat für die Qualitätsmessung, wenn mindestens eine der Teilelektroden ganz unter dem Flüssigkeitsniveau liegt.

Vereinfachte Varianten der im Zusammenhang mit den Figuren 2 und 3 beschriebenen Ausführungsform der erfindungsgemässen Anordnung bestehen darin, dass unter der Annahme, Qualität und Temperatur im Überlauf müssten nicht kontrolliert werden, eine Anordnung mit nur einer Teilelektrode verwendet wird und ihr Messsignal mit dem reduzierten Messwert des Sensors RS verglichen und daraus die Niveauhöhe N_{K} bestimmt wird. Diese vereinfachte Variante hat dann dieselbe Funktion wie die im Zusammenhang mit der Figur 1 beschriebene Variante.

**Figur 4** zeigt eine beispielhafte Ausführungsform der Anordnung eines Messelementes im Überlauf der Kammer, von der nur die Einlaufpartie im Schnitt mit einem einlaufenden Faden F dargestellt ist. Sie weist zwei Elektroden 41 und 42 auf, von denen mindestens die eine in der beschriebenen Weise in mindestens drei Teilelektroden unterteilt sein kann. Die Elektroden sind in einem isolierenden Kunststoffteil 43 eingegossen, der unten im Überlauf derart positioniert ist, dass die Elektroden bis zu einer Höhe reichen, die einem höchsten Flüssigkeitsniveau im Überlauf entspricht. Die Elektroden sind durch Signalleitungen 41.1 und 42.1 mit der Steuereinheit verbunden.

**Figur 5** zeigt in derselben Darstellungsweise wie Figur 4 eine weitere Variante der Anordnung des Messelementes im Überlauf der Streckkammer. Ein isolierender Kunststoffteil 53 ist oberhalb des höchstmöglichen Niveaus Nₘₐₓ angeordnet und zwei Elektroden 51 und 52 ragen von oben in die Flüssigkeit, derart, dass sie bis gerade unter ein minimal tolerierbares Niveau Nₘᵢₙ reichen. Bei einem Niveau N für normalen Betrieb sind die Elektroden teilweise eingetaucht.

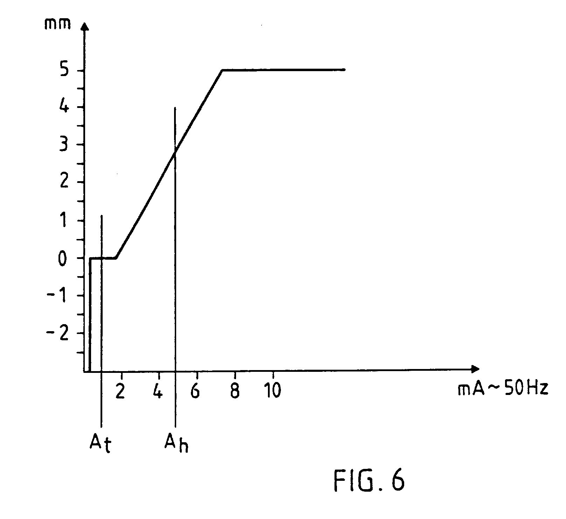

**Figur 6** zeigt den Verlauf des Messsignales bei variierendem Flüssigkeitsniveau für eine Anordnung gemäss Figur 5, mit einem Elektrodenquerschnitt von 1,6 x 0,8mm, einem Elektrodenabstand von 8mm, einer Steuerspannung von 10V/50Hz und einer Kammerflüssigkeit aus Wasser mit 10% Spin-Finish. Das Messelement ist derart angeordnet, dass die Elektroden bis 2mm über die Überlaufgrundfläche reichen und der isolierende Teil mindestens 5mm über dieser Grundfläche angeordnet ist. Das Toleranzband ist zwischen 1 und 5mA angesetzt, was einem Niveau zwischen 2mm (Niveau auf Eintauchtiefe der Elektroden, untere Alarmgrenze Aₜ) und etwas unter 5mm (Niveau auf 3mm Eintauchtiefe der Elektroden, obere Alarmgrenze Aₕ) entspricht.

## Patentansprüche

1. Streckkammeranordnung zum Strecken eines Filamentfadens mit einer Streckkammer (1) mit Überlauf (1.1) und einem Kammer-flüssigkeitskreislauf, bestehend aus einer Zuführung (2) in die Kammer, einer Abführung aus der Kammer (3), einem Tank (4) und einem Pumpmittel (5), **dadurch gekennzeichnet**, dass sie im Bereiche des Überlaufs der Kammer ein Messelement (6) zur Messung des elektrischen Widerstandes der Kammerflüssigkeit aufweist, das derart angeordnet ist, dass mindestens eine Messelektrode vom Flüssigkeitsniveau in einen benetzten und einen nicht-benetzten Teil geteilt wird, sodass das Messsignal von der Höhe des Flüssigkeitsniveaus abhängig ist.

2. Streckkammeranordnung nach Anspruch 1, **dadurch gekennzeichnet**, dass das Messelement (6) im Bereiche des Kammerüberlaufes mit einem Toleranzbandumsetzer (8.1) verbunden ist, der das Messsignal des Messelementes mit einem vorgebbaren Toleranzband vergleicht und bei einem Messsignal, das nicht im Toleranzband liegt, ein Alarmmittel (9) und/oder eine Fadenschere (10) aktiviert.

3. Streckkammeranordnung nach einem der Anspräche 1 oder 2, **dadurch gekennzeichnet**, dass das Messelement (6) im Überlauf der Kammer derart angeordnet ist, dass seine Elektroden (51, 52) von oben unter das Flüssigkeitsniveau des Überlaufes ragen.

4. Streckkammeranordnung nach einem der Ansprüche 1 oder 2, **dadurch gekennzeichnet**, dass das Messelement (6) im Überlauf der Kammer derart angeordnet ist, dass seine Messelektroden (41, 42) von unten über das Niveau des Überlaufes ragen.

5. Streckkammeranordnung nach Anspruch 1, **dadurch gekennzeichnet**, dass mindestens eine Elektrode des Messelementes (KS) im Überlauf der Kammer in Teilelektroden (KS.1, KS.2, KS.3) aufgeteilt ist, dass jede Teilelektrode mit einer Steuereinheit (30) verbunden ist und dass die Steuereinheit derart ausgelegt ist, dass sie die Messsignale der Teilelektroden mit vorgebbaren Toleranzbändern vergleichen und entsprechende Alarm- und/oder Steuersignale generieren kann.

6. Streckkammeranordnung gemäss Anspruch 1, **dadurch gekennzeichnet**, dass im Tank ein weiteres Messelement (7) zur Messung des elektrischen Widerstandes der Kammerflüssigkeit derart angeordnet ist, dass sein Messsignal nicht von der Füllhöhe des Tankes abhängig ist.

7. Streckkammeranordnung nach Anspruch 6, **dadurch gekennzeichnet**, dass das Messelement (7) im Tank mit einem Toleranzbandumsetzer (8.2) verbunden ist, der den Messwert mit einem vorgebbaren Toleranzband vergleicht und, wenn das Messsignal ausserhalb des Toleranzbandes liegt, ein Alarmmittel (9) und/oder die Steuerung eines Dosierventiles (4.3) einer Wasserzuleitung (4.2) zum Tank (4) aktiviert.

8. Streckkammeranordnung nach Anspruch 6, **dadurch gekennzeichnet**, dass mindestens eine Elektrode des Messelementes (KS) im Überlauf der Kammer in Teilelektroden (KS.1, KS.2, KS.3) aufgeteilt ist, dass jede Teilelektrode mit einer Steuereinheit (30) verbunden ist und dass das Messelement (RS) im Tank (4) mit derselben Steuereinheit verbunden ist.

9. Streckkammeranordnung nach Anspruch 8, **dadurch gekennzeichnet**, dass die Steuereinheit (30) derart ausgelegt ist, dass sie den Messwert des Messelementes (RS) im Tank mit einem Toleranzband vergleichen und in eine mit den Messwerten der Teilelektroden (KS,1, KS.2, KS.3) des Messelementes im Überlauf vergleichbare Form bringen kann und dass sie diese Vergleiche in einer vorgegebenen Reihenfolge durchführen und entsprechende Alarm- und/oder Steuersignale generieren kann.

10. Streckkammeranordnung zum Strecken eines Filamentfadens mit einer Streckkammer (1) mit Überlauf (1.1), einer Flüssigkeitszuführung (2) in die Kammer und einer Flüssigkeitsabführung aus der Kammer (3) und einem Pumpmittel (5), dadurch gekennzeichnet, dass im Bereiche des Überlaufs der Kammer ein Messelement (6) zur Messung des elektrischen Widerstandes der Kammerflüssigkeit aufweist, welches derart angeordnet ist, dass mindestens eine Messelektrode vom Flüssigkeitsniveau in einen benetzten und einen nicht-benetzten Teil geteilt wird, sodass das Messsignal von der Höhe des Flüssigkeitsniveaus und der Qualität der Flüssigkeit abhängig ist, wobei für die Messung der Qualität mindestens eine Elektrode des Messelementes (KS) im Überlauf der Kammer in Teilelektroden (KS.1, KS.2, KS.3) aufgeteilt ist und mindestens eine der Teilelektroden ganz unter dem Flüssigkeitsniveau liegt.

11. Streckkammeranordnung nach Anspruch 10, dadurch gekennzeichnet, dass jede Teilelektrode mit einer Steuereinheit (30) verbunden ist und dass die Steuereinheit derart ausgelegt ist, dass sie die Messsignale der Teilelektroden mit vorgebbaren Toleranzbändern vergleichen und entsprechende Alarm- und/oder Steuersignale generieren kann.

## Claims

1. A stretching chamber arrangement for stretching a filament yarn with a stretching chamber (1) comprising an overflow (1.1) and a chamber liquid circulation, consisting of a feed conduit (2) leading to the chamber, a return conduit (3) leading away from the chamber, a tank (4) and a pumping means (5), characterized in that said arrangement comprises in the area of the overflow of the chamber a measuring element (6) for measuring the electric resistance of the chamber liquid, which element is arranged in such a way that at least one measuring electrode is divided by the liquid level into a wetted and non-wetted part, so that the measuring signal depends on the height of the liquid level.

2. A stretching chamber arrangement as claimed in claim 1, characterized in that in the area of the chamber overflow the measuring element (6) is connected with a tolerance band converter (8.1) which compares the measuring signal of the measuring element with a predeterminable tolerance band and which activates an alarm means (9) and/or a yarn cutter (10) whenever a measuring signal occurs which does not lie within the tolerance band.

3. A stretching chamber arrangement as claimed in one of the claims 1 or 2, characterized in that the measuring element (6) is arranged in the overflow of the chamber in such a way that its electrodes (51, 52) project from above below the liquid level of the overflow.

4. A stretching chamber arrangement as claimed in one of the claims 1 or 2, characterized in that the measuring element (6) in the overflow of the chamber is arranged in such a way that its measuring electrodes (41, 42) project from below above the liquid level of the overflow.

5. A stretching chamber arrangement as claimed in claim 1, characterized in that in the overflow of the chamber at least one electrode of the measuring element (KS) is subdivided into partial electrodes (KS.1, KS.2, KS.3), each partial electrode is connected with a control unit (30) and the control unit is arranged in such a way that it can compare the measuring signals of the partial electrodes with predeterminable tolerance bands and generate respective alarm and/or control signals.

6. A stretching chamber arrangement as claimed in claim 1, characterized in that a further measuring element (7) for measuring the electric resistance of the chamber liquid is arranged in the tank in such a way that its measuring signal does not depend on the filling level of the tank.

7. A stretching chamber arrangement as claimed in claim 6, characterized in that the measuring element (7) in the tank is connected with a tolerance band converter (8.2) which compares the measured value with a predeterminable tolerance band and, if the measuring signal lies outside of the tolerance band, activates an alarm means (9) and/or the control unit of a dosaging valve (4.3) of a water supply line (4.2) leading to the tank (4).

8. A stretching chamber arrangement as claimed in claim 6, characterized in that at least one electrode of the measuring element (KS) is subdivided into partial electrodes (KS.1, KS.2, KS.3) in the overflow of the chamber, each partial electrode is connected with a control unit (30) and the measuring element (RS) in the tank (4) is connected with the same control unit.

9. A stretching chamber arrangement as claimed in claim 8, characterized in that the control unit (30) is arranged in such a way that it can compare the measured value of the measuring element (RS) in the tank with a tolerance band and that it can bring it into such a form that it can be compared with the measured values of the partial electrodes (KS.1, KS.2, KS.3) of the measuring element in the overflow and that it can carry out said comparisons in a predetermined sequence and generate respective alarm and/or control signals.

10. A stretching chamber arrangement for stretching a filament yarn with a stretching chamber (1) comprising an overflow (1.1), a liquid supply (2) into the chamber and a liquid discharge from the chamber (3) and a pumping means (5), characterized in that said arrangement comprises in the area of the overflow of the chamber a measuring element (6) for measuring the electric resistance of the chamber liquid which is arranged in such a way that at least one measuring electrode is divided by the liquid level into a wetted and non-wetted part, so that the measuring signal depends on the height of the liquid level and the quality of the liquid, with at least one electrode of the measuring element (KS) being subdivided into partial electrodes (KS.1, KS.2, KS.3) in the overflow of the chamber for measuring the quality and at least one of the partial electrodes being entirely below the liquid level.

11. A stretching chamber arrangement as claimed in claim 10, characterized in that each partial electrode is connected with a control unit (30) and the control unit is arranged in such a way that it can compare the measuring signals of the partial electrodes with predeterminable tolerance bands and generate respective alarm and/or control signals.

## Revendications

1. Arrangement de chambre d'étirage servant à étirer un fil de filaments, avec une chambre d'étirage (1) possédant un trop-plein (1.1), et un circuit de liquide de chambre constitué par une amenée (2) dans la chambre, une sortie (3) hors de la chambre, un réservoir (4) et un moyen de pompage (5),
caractérisé par le fait que,
dans la zone du trop-plein de la chambre, l'arrangement possède un élément de mesure (6) servant à mesurer la résistance électrique du liquide de chambre, qui est disposé de telle sorte qu'au moins une électrode de mesure est divisée, par le niveau de liquide, en une partie mouillée et une partie non mouillée, de sorte que le signal de mesure est dépendant de la hauteur du niveau de liquide.

2. Arrangement de chambre d'étirage selon revendication 1,
caractérisé par le fait que,
dans la zone du trop-plein de la chambre, l'élément de mesure (6) est relié avec un convertisseur de bande de tolérance (8.1) qui compare le signal de mesure de l'élément de mesure avec une bande de tolérance pouvant être prédéterminée, et active un moyen d'alarme (9) et/ou des ciseaux de fil (10), lorsqu'un signal de mesure ne se situe pas dans la bande de tolérance.

3. Arrangement de chambre d'étirage selon une des revendications 1 ou 2,
caractérisé par le fait que
l'élément de mesure (6) est disposé dans le trop-plein de la chambre, de telle façon que ses électrodes (51, 52) pénètrent, depuis le haut, sous le niveau de liquide du trop-plein.

4. Arrangement de chambre d'étirage selon une des revendications 1 ou 2,
caractérisé par le fait que
l'élément de mesure (6) est disposé dans le trop-plein de la chambre, de telle façon que ses électrodes (41, 42) pénètrent, depuis le bas, au-dessus du niveau de liquide du trop-plein.

5. Arrangement de chambre d'étirage selon revendication 1,
caractérisé par le fait
qu'au moins une électrode de l'élément de mesure (KS), situé dans le trop-plein de la chambre, est divisée en électrodes partielles (KS.1, KS.2, KS.3), que chaque électrode partielle est reliée avec une unité d'asservissement (30), et que l'unité d'asservissement est conçue de telle sorte qu'elle peut comparer les signaux de mesure des électrodes partielles avec des bandes de tolérance pouvant être prédéterminées, et générer des signaux d'alarme et/ou d'asservissement correspondants.

6. Arrangement de chambre d'étirage selon revendication 1,
caractérisé par le fait
qu'un autre élément de mesure (7) servant à mesurer la résistance électrique du liquide de chambre, est disposé de telle sorte dans le réservoir que son signal de mesure n'est pas dépendant de la hauteur de remplissage du réservoir.

7. Arrangement de chambre d'étirage selon revendication 6,
caractérisé par le fait que
l'élément de mesure (7) disposé dans le réservoir est relié avec un convertisseur de bande de tolérance (8.2) qui compare la valeur de mesure avec une bande de tolérance pouvant être prédéterminée, et, lorsque le signal de mesure se situe en dehors de la bande de tolérance, active un moyen d'alarme (9) et/ou l'asservissement d'une vanne de dosage (4.3) d'une amenée d'eau (4.2) vers le réservoir (4).

8. Arrangement de chambre d'étirage selon revendication 6,
caractérisé par le fait
qu'au moins une électrode de l'élément de mesure (KS), situé dans le trop-plein de la chambre, est divisée en électrodes partielles (KS.1, KS.2, KS.3), que chaque électrode partielle est reliée avec une unité d'asservissement (30), et que l'élément de mesure (RS), disposé dans le réservoir (4), est relié avec la même unité d'asservissement.

9. Arrangement de chambre d'étirage selon revendication 8,
caractérisé par le fait que
l'unité d'asservissement (30) est conçue de telle sorte qu'elle peut comparer la valeur de mesure de l'élément de mesure (RS) disposé dans le réservoir avec une bande de tolérance, et l'amener en une forme pouvant être comparée avec les valeurs de mesure des électrodes partielles (KS.1, KS.2, KS.3) de l'élément de mesure situé dans le trop-plein, et qu'elle peut réaliser ces comparaisons dans un ordre prédéterminé, et générer des signaux d'alarme et/ou d'asservissement correspondants.

10. Arrangement de chambre d'étirage servant à étirer un fil de filaments, avec une chambre d'étirage (1) possédant un trop-plein (1.1), une amenée de liquide (2) dans la chambre, et une sortie de liquide (3) hors de la chambre, et un moyen de pompage (5),
caractérisé par le fait que,
dans la zone du trop-plein de la chambre, l'arrangement possède un élément de mesure (6) servant à mesurer la résistance électrique du liquide de chambre, qui est disposé de telle sorte qu'au moins une électrode de mesure est divisée, par le niveau de liquide, en une partie mouillée et une partie non mouillée, de sorte que le signal de mesure est dépendant de la hauteur du niveau de liquide et de la qualité du liquide, et où, pour la mesure de la qualité, au moins une électrode de l'élément de mesure (KS), situé dans le trop-plein de la chambre, est divisée en électrodes partielles (KS.1, KS.2, KS.3), et au moins une des électrodes partielles est située entièrement sous le niveau du liquide.

11. Arrangement de chambre d'étirage selon revendication 10,
caractérisé par le fait que
chaque électrode partielle est reliée avec une unité d'asservissement (30), et que l'unité d'asservissement est conçue de telle sorte qu'elle peut comparer les signaux de mesure des électrodes partielles avec des bandes de tolérance pouvant être prédéterminées, et générer des signaux d'alarme et/ou d'asservissement correspondants.
